# EUROPEAN PATENT APPLICATION

(11) **EP 4 537 836 A1**
(43) Date of publication of application: **16.04.2025**
(21) Application number: 22945580.3
(22) Date of filing: 21.11.2022
(51) Int. Cl.: A61K 35/744, A61K 9/70, A61P 1/02, C12N 1/04, C12R 1/225

(54) **PROBIOTIC PATCH FOR PREVENTING AND TREATING ORAL ULCER, METHOD FOR PREPARING SAME AND USE THEREOF**

(30) Priority: 10.06.2022 CN 202210652284
(71) Applicant: Inner Mongolia Yili Industrial Group Co. Ltd., Hohhot, Inner Mongolia 010000 (CN)
(72) Inventor: LIU, Fudong, Hohhot, Inner Mongolia 010000 (CN); HUNG, Wei-Lian, Hohhot, Inner Mongolia 010000 (CN); LIU, Wei-Hsien, Hohhot, Inner Mongolia 010000 (CN); ZHAO, Wen, Hohhot, Inner Mongolia 010000 (CN)
(74) Representative: Dehns
(86) International application number: PCT/CN2022/133142
(87) International publication number: WO 2023/236452

(57) **Abstract**

Provided is use of *Lactobacillus paracasei* in preparing a medicament for preventing and treating oral ulcer. The *Lactobacillus paracasei* comprises *Lactobacillus paracasei* ET-22 cells and/or *Lactobacillus paracasei* ET-22 extracellular metabolites. *Lactobacillus paracasei* ET-22 is a strain with deposition number CGMCC No. 15077. Also provided are a probiotic composition, a method for preparing same, and use thereof. The composition comprises *Lactobacillus paracasei* ET-22 cells and/or *Lactobacillus paracasei* ET-22 extracellular metabolites, and further comprises sodium carboxymethyl cellulose, propolis, borneol, a lubricant, glycerol, and Tween-20. Further provided is a probiotic patch for preventing and treating oral ulcer, a method for preparing same, and use thereof.

## Description

### Technical Field

The present invention relates to a probiotic patch, a method for preparing same, and the use thereof, in particular to the use of *Lactobacillus paracasei* ET-22 in preparing a medicament for preventing and treating oral ulcer, a probiotic composition comprising *Lactobacillus paracasei* ET-22, and a probiotic patch prepared from the composition, a method for preparing same, and the use thereof.

### Background Art

Oral ulcer, commonly known as "mouth sore", is a common symptom of ulcerative lesions that occur in the oral mucosa. Oral ulcer occurs as a result of a combination of factors, including local trauma, mental stress, food, medicaments, malnutrition, altered hormone levels, and deficiencies in vitamins or trace elements.

A variety of probiotics are known in the art to help treat oral ulcer. CN 109985179 A discloses a composition for treating oral ulcer, which composition comprises the following raw materials in parts by weight: 1000-15000 parts of fresh asparagus, 0.1-10 parts of *Lactobacillus salivarius,* 0.1-20 parts of *Bifidobacterium lactis,* 0.1-20 parts of *Lactobacillus paracasei,* 0.1-50 parts of *Indigo Naturalis,* and 10-1000 parts of starch; and the composition can serve to supplement trace elements required by human body, to have the effect of intrinsic immunomodulation and oral environment improvement for patients with oral ulcer, and to enhance the body's immunity, and is prepared into an oral medicament such as a pill, a powder, a capsule, a tablet or a granule when particularly used for promoting sore healing. CN 113425831 A discloses a probiotic composition for treating oral ulcer, a method for preparing same, and the use thereof, wherein the probiotic composition comprises the following raw materials in parts by weight: 40-60 parts of *Borojoa patinoi* powder, 30-60 parts of *Lithocarpus litseifolius (Hance) Chun,* 10-20 parts of probiotics, 1-5 parts of *Chimonanthus salicifolius,* 1-5 parts of mannose oligosaccharides, 1-5 parts of leaves of *Linderae radix,* and 1-5 parts of colostrum basic protein; and according to the composition, *Borojoa patinoi* powder and *Lithocarpus litseifolius (Hance) Chun* having an antioxidant effect are used to achieve the effect of treating oral ulcer, the amount of probiotics can be reduced, mannose oligosaccharides can also promote the growth of probiotics to play a coordinating role among the probiotics, and Chimonanthus salicifolius, rice bran fatty alcohol, the leaves of *Linderae radix* and colostrum basic protein contained therein all have an effect of treating oral ulcer so as to prevent and improving oral ulcer, and the composition is prepared into a spray when particularly used.

For the above-mentioned probiotic-containing medicaments for treating oral ulcer in the prior art, probiotics themselves contribute moderately to the treatment of oral ulcer, and oral medicaments have disadvantages such as wide action sites, large dosages, and low utilization rate of medicaments. Since water deprivation and fasting for a certain period of time are required after spraying, there are many inconveniences when using the medicaments.

### Summary of the Invention

An objective of the present invention is to provide a probiotic for preventing and treating oral ulcer.

Another objective of the present invention is to provide the use of *Lactobacillus paracasei* ET-22 in preparing a medicament for preventing and treating oral ulcer.

Another objective of the present invention is to provide a probiotic composition comprising *Lactobacillus paracasei* ET-22.

Another objective of the present invention is to provide a probiotic patch prepared from the composition.

Another objective of the present invention is to provide a method for preparing a probiotic patch.

Another objective of the present invention is to provide the use of a probiotic composition or a patch.

The inventors of the present invention have found in studies that *Lactobacillus paracasei* ET-22 is a probiotic that can prevent and treat oral ulcer, and the bacterial cells and/or extracellular metabolites thereof can significantly prevent and treat oral ulcer. Further, the *Lactobacillus paracasei* ET-22 cells and/or *Lactobacillus paracasei* ET-22 extracellular metabolites are prepared into a probiotic patch, which can effectively sterilize oral ulcer parts and effectively prevent secondary infection of wounds.

In the present invention, the preventing and treating includes preventing and/or treating.

Therefore, in one aspect, the present invention provides the use of *Lactobacillus paracasei* in preparing a medicament for treating oral ulcer, wherein the *Lactobacillus paracasei* comprises *Lactobacillus paracasei* ET-22 cells and/or *Lactobacillus paracasei* ET-22 extracellular metabolites, and the *Lactobacillus paracasei* ET-22 is a strain with deposition number CGMCC No. 15077.

The *Lactobacillus paracasei* ET-22 strain with deposition number CGMCC No. 15077 is a biological material published in CN 110964653 A and is available to the public.

According to a particular embodiment of the present invention, in the present invention, the *Lactobacillus paracasei* ET-22 cells are *Lactobacillus paracasei* ET-22 live cells and/or *Lactobacillus paracasei* ET-22 inactivated cells.

According to a particular embodiment of the present invention, in the present invention, the *Lactobacillus paracasei* ET-22 extracellular metabolites are prepared according to the following method comprising:
removing the bacterial cells from an incubation solution of *Lactobacillus paracasei* ET-22 to obtain the *Lactobacillus paracasei* ET-22 extracellular metabolites; preferably, the incubation solution is obtained by incubating the *Lactobacillus paracasei* ET-22 in water.

Preferably, *Lactobacillus paracasei* ET-22 cells are dissolved in water at a concentration of 1 to 5 × 10⁹ CFU/mL, and stirred and incubated at 37°C under 200 to 500 rpm for 2-4 hours, and after the incubation is completed, the bacterial cells are removed by centrifugation to obtain the *Lactobacillus paracasei* ET-22 extracellular metabolites.

According to a particular embodiment of the present invention, in the present invention, the oral ulcer includes oral ulcer caused by pathogenic bacteria, oral ulcer caused by oxidative damage, or oral ulcer caused by immunocompromise.

In another aspect, the present invention further provides a probiotic composition, comprising: *Lactobacillus paracasei* ET-22 cells and/or *Lactobacillus paracasei* ET-22 extracellular metabolites, wherein the *Lactobacillus paracasei* ET-22 is a strain with deposition number CGMCC No. 15077.

According to a particular embodiment of the present invention, in the present invention, the probiotic composition further comprises: sodium carboxymethyl cellulose, propolis, borneol, a lubricant, glycerol, and Tween-20, wherein preferably, the probiotic composition comprises the following components in parts by weight:
15-20 parts of *Lactobacillus paracasei* ET-22 cells and/or *Lactobacillus paracasei* ET-22 extracellular metabolites;
10-20 parts of sodium carboxymethyl cellulose;
20-40 parts of propolis;
2-5 parts of borneol;
2-5 parts of lubricant;
8-12 parts of glycerol;
and 1-3 parts of Tween-20.

According to a particular embodiment of the present invention, the probiotic composition of the present invention comprises the following components in parts by weight:
18-20 parts of *Lactobacillus paracasei* ET-22 cells and/or *Lactobacillus paracasei* ET-22 extracellular metabolites;
14-20 parts of sodium carboxymethyl cellulose;
25-35 parts of propolis;
2-5 parts of borneol;
2-5 parts of lubricant;
8-12 parts of glycerol;
and 1-3 parts of Tween-20.

According to a particular embodiment of the present invention, in the probiotic composition of the present invention, the *Lactobacillus paracasei* ET-22 cells are *Lactobacillus paracasei* ET-22 live cells and/or *Lactobacillus paracasei* ET-22 inactivated cells.

According to a particular embodiment of the present invention, in the probiotic composition of the present invention, the *Lactobacillus paracasei* ET-22 extracellular metabolites are prepared according to the following method comprising:
*Lactobacillus paracasei* ET-22 cells are dissolved in water at a concentration of 1 to 5 × 10⁹ CFU/mL, and stirred and incubated at 37°C under 200 to 500 rpm for 2-4 hours, and after the incubation is completed, the bacterial cells are removed by centrifugation to obtain the *Lactobacillus paracasei* ET-22 extracellular metabolites.

According to a particular embodiment of the present invention, in the probiotic composition of the present invention, the lubricant can be liquid paraffin or other lubricants commonly used in oral patches.

In another aspect, the present invention further provides the use of the probiotic composition in preparing a composition for preventing and treating oral ulcer.

According to a particular embodiment of the present invention, the oral ulcer includes oral ulcer caused by pathogenic bacteria, oral ulcer caused by oxidative damage, or oral ulcer caused by immunocompromise. In some particular embodiments of the present invention, *Lactobacillus paracasei* ET-22 live cells can significantly prevent and treat oral ulcer caused by pathogenic bacteria or oxidative damage. In some particular embodiments of the present invention, the application of *Lactobacillus paracasei* ET-22 inactivated cells can significantly prevent and treat oral ulcer caused by immunocompromise. In some particular embodiments of the present invention, the *Lactobacillus paracasei* ET-22 extracellular metabolites have a significant effect of preventing and treating oral ulcer.

According to some particular embodiments of the present invention, the composition for preventing and treating oral ulcer of the present invention is a pharmaceutical composition. Preferably, the pharmaceutical composition is a patch.

According to some particular embodiments of the present invention, the composition for preventing and treating oral ulcer of the present invention can be a general cosmetic.

In another aspect, the present invention further provides a method for preparing a probiotic patch, wherein the method comprises:
(1) a step of uniformly mixing *Lactobacillus paracasei* ET-22 cells and/or *Lactobacillus paracasei* ET-22 extracellular metabolites, propolis, sodium carboxymethyl cellulose and a lubricant to obtain a mixed system A;
(2) a step of heating and uniformly mixing borneol, glycerol and Tween-20 in a water insulation manner to obtain a mixed system B;
(3) a step of uniformly mixing the mixed system A and the mixed system B;
and (4) a step of performing drying treatment.

It should be noted that reference numerals (1) and (2) for the steps of the preparation method of the present invention do not indicate that the two steps must be performed in the order described, and in actual operation, step (2) can be performed first and then step (1) can be performed, or both steps are performed simultaneously.

In another aspect, the present invention further provides a probiotic patch, which is prepared from the composition of the present invention, or prepared by the above-mentioned method for preparing the probiotic patch.

In another aspect, the present invention further provides a method for treating oral ulcer, which method comprises administering to a subject an effective amount of the composition and/or the probiotic patch, wherein the subject includes a mammal or a human.

In summary, the present invention provides the use of *Lactobacillus paracasei* ET-22 and/or extracellular metabolites thereof in preventing and treating oral ulcer, and provides the resulting probiotic patch. The present invention proves through animal models that the *Lactobacillus paracasei* ET-22 live cells can significantly prevent and treat oral ulcer caused by pathogenic bacteria or oxidative damage; the application of the *Lactobacillus paracasei* ET-22 inactivated cells can significantly prevent and treat oral ulcer caused by immunocompromise; and the *Lactobacillus paracasei* ET-22 extracellular metabolites have good effects when used for the prevention and/or treatment of oral diseases. The probiotic patch of the present invention has good adhesion to the oral mucosa; due to the addition of borneol, the probiotic patch also has the effects of clearing heat and removing toxicity, detumescence and relieving pain; and the probiotic patch can effectively sterilize oral ulcer parts and effectively prevent secondary infection of wounds.

### Brief Description of the Drawings

FIG. 1 shows ICR mouse tongue injury scores 48 h after modeling in the prevention group in Example 2.
FIG. 2 shows ICR mouse tongue injury scores 72 h after modeling in the treatment group in Example 2.
FIG. 3 shows HE staining of tongue dorsum lesion sections of mice in the blank group, model group and probiotic group 48 h after modeling in the prevention group in Example 2; where the photographs in A show the papillary process of epithelial cells in tongue tissue, and the photographs in B show tissue hyperplasia.
FIG. 4 shows HE staining photographs of typical oral mucosal tissue sections of hamsters 48 h after modeling in the prevention group in Example 2; where white arrows point to inflammatory cell infiltration, black arrows point to damage and shedding of mucosal epithelium, and gray arrows point to capillary filling and dilation and gland hyperplasia (scale bar: 200 µm).
FIG. 5 shows immunohistochemical photographs of typical oral ulcer mucosal tissues of hamsters 48 h after modeling in the prevention group of Example 2 (scale bar: 100 µm).
FIG. 6 shows the levels of LXA4 and PGE2 in the oral ulcer mucosal tissue and the levels of pro-inflammatory cytokines IL-6 and IL-1β in sera of hamsters in Example 2.
FIG. 7 shows SOD activity, and GSH and MDA levels in sera of hamsters in Example 2.
FIG. 8 shows the composition of oral bacteria in hamsters at the phylum level in Example 2.
FIG. 9 shows bacterial genera with significant differences in the oral cavity of hamsters in Example 2.
FIG. 10 shows changes in the biomass of simulated tooth surface biofilms under different probiotic interventions in Example 3.
FIG. 11 shows changes in the biomass structure of simulated tooth surface biofilms under different probiotic interventions by scanning electron microscope in Example 3.
FIG. 12 shows changes in the biomass structure of simulated tooth surface biofilms under different probiotic interventions by confocal laser scanning microscope in Example 3.
FIG. 13 shows changes in the thickness of simulated tooth surface biofilms under different probiotic interventions in Example 3.

### Detailed Description of Embodiments

In order to understand the technical features, objectives and beneficial effects of the present invention more clearly, the technical solutions of the present invention are described in detail below in conjunction with specific examples. It should be understood that these examples are only used to illustrate the present invention rather than limit the scope of the present invention. In the examples, the reagent raw materials are commercially available, and experimental methods without specifying specific conditions are conventional methods and conditions well known in the art, or are conducted according to conditions suggested by an instrument manufacturer.

Unless specifically defined otherwise, all the technical and scientific terms used herein have the same meanings as commonly understood by those of ordinary skill in the relevant art.

In various examples of the present invention, the *Lactobacillus paracasei* ET-22 extracellular metabolites are prepared according to the following method comprising: *Lactobacillus paracasei* ET-22 is inoculated into a culture medium, and cultured to obtain a bacterial liquid, and the ET-22 liquid is centrifuged to obtain *Lactobacillus paracasei* ET-22 cells (sludge); and
the obtained *Lactobacillus paracasei* ET-22 cells are dissolved in water at a concentration of 1 to 5 × 10⁹ CFU/mL, and stirred and incubated at 37°C under 200 to 500 rpm for 3 hours, and after the incubation is completed, the bacterial cells are removed by centrifugation so as to obtain the *Lactobacillus paracasei* ET-22 extracellular metabolites.

### Example 1

This example provides a probiotic patch for treating oral ulcer. The raw materials for preparing the probiotic patch comprise *Lactobacillus paracasei* ET-22 cells and/or *Lactobacillus paracasei* ET-22 extracellular metabolites, sodium carboxymethyl cellulose, propolis, borneol, a lubricant, glycerol, and Tween-20 in parts by weight:
18 parts of *Lactobacillus paracasei* ET-22 cells and/or *Lactobacillus paracasei* ET-22 extracellular metabolites;
15 parts of sodium carboxymethyl cellulose;
30 parts of propolis;
5 parts of borneol;
5 parts of lubricant;
10 parts of glycerol;
and 2 parts of Tween-20.

The method for preparing the probiotic patch comprises the following steps:
(1) mixing *Lactobacillus paracasei* ET-22 cells and/or *Lactobacillus paracasei* ET-22 extracellular metabolites, propolis, sodium carboxymethyl cellulose and lubricant according to the above-mentioned weight ratio in parts to obtain a mixed system A;
(2) placing borneol, glycerol and Tween-20 in a container according to the above-mentioned weight ratio in parts for heating in a water insulation manner to obtain a mixed system B;
and (3) uniformly mixing the mixed system A and the mixed system B, and performing drying treatment to prepare the probiotic patch for treating oral ulcer.

### Example 2 Effect of Lactobacillus paracasei ET-22 in preventing and treating oral ulcer

### 1. Experimental animal models

### 1.1 Models of oral ulcer caused by immunocompromise

**Animal models in the prevention group:** To induce oral candidiasis in mice, a previously described protocol was used in the present invention with some minor modifications. After the adaptation period, the mice in the experimental group were subjected to probiotic intervention (addition of probiotic to drinking water at 10⁹ CFU/ml) for 18 days, while the mice in the blank group and model group received normal drinking water. On day 15, the mice were given 15 mg/ml tetracycline hydrochloride via drinking water, and simultaneously an immunosuppressive state was induced by subcutaneously injecting prednisolone (100 mg/kg). On day 16, the mice in the model group and experimental group were infected with *Candida albicans* CGMCC 2.4122, wherein during the infection, 4% trichloroacetaldehyde hydrate (10 ml/kg) was used for anesthesia, then various parts of the oral cavity were infected with *Candida albicans* at 1.0 × 10⁹ CFU/ml, and a *Candida albicans-*impregnated cotton swab was placed on the tongue dorsum for 15 minutes. Mice were euthanized 48 hours after the surgery.

**Animal models in the treatment group:** To induce oral candidiasis in mice, a previously described protocol was used in the present invention with some minor modifications. After the adaptation period of mice, the mice were given 15 mg/ml tetracycline hydrochloride via drinking water, and simultaneously an immunosuppressive state was induced by subcutaneously injecting prednisolone (100 mg/kg). On day 2, the mice in the model group and experimental group were infected with *Candida albicans* CGMCC 2.4122, wherein during the infection, 4% trichloroacetaldehyde hydrate (10 ml/kg) was used for anesthesia, then various parts of the oral cavity were infected with *Candida albicans* at 1.0 × 10⁹ CFU/ml, and a *Candida* albicans-impregnated cotton swab was placed on the tongue dorsum for 15 minutes. 24 hours after infection (day 3), the mice in the probiotic intervention group were subjected to one probiotic application intervention every 12 hours, for a total of four applications, wherein the application method was the same as the application method during the infection. The mice were euthanized 72 hours after the surgery (day 5).

### 1.2 Golden hamster models of oral ulcer caused by oxidative damage

Six-week old male LVG Golden Syrian Hamsters (body weight 110 ± 20 g/hamster) were purchased from Beijing Vital River Laboratory Animal Technology Co., Ltd., and were housed under 12 h light/dark alternation, with a temperature maintained at 20°C ± 2°C and a humidity maintained at 45%-50%. After one week of domestication in the rearing chamber, the hamsters were randomly divided into 10 groups, normal control group, model group, ET-22 living cell group, ET-22 inactivated cell group, ET-22 extracellular metabolite group, K12 living cell group, HN019 living cell group, DSM17938 living cell group, AP32 living cell group, and vitamin C positive control group (VC group), with 7 hamsters in the normal control group and 10 hamsters in each of the other groups. The hamsters in the normal control group and model group were given intragastrically 1 ml of physiological saline every day, the hamsters in the ET-22 living cell group were given intragastrically 1 ml of ET-22 living cell solution with a concentration of 10⁹ CFU/ml every day, the hamsters in the ET-22 inactivated cell group and ET-22 extracellular metabolite group were given intragastrically 1 ml of ET-22 inactivated cells and ET-22 extracellular metabolites with a corresponding concentration, respectively, and the hamsters in the VC group were given intragastrically 1 ml of vitamin C solution with a concentration of 20 mg/ml every day. Oral ulcer modeling was performed 15 days after intragastric administration. The hamsters were anesthetized by intraperitoneal injection of 10% chloral hydrate (injection volume: 0.3-0.5% of body weight), and then 0.25 ml of PBS buffer (pH = 7.4) was injected into the cheek pouch of the hamsters in the normal control group and 0.25 ml of methyl viologen (10 mmol/L, dissolved in PBS) was injected into the cheek pouch of the hamsters in the remaining 5 groups. From the day of injection to the sacrifice of the hamsters, intragastric administration intervention was performed on the hamsters in each group every day. On day 4 after modeling, the hamsters were anesthetized, the blood was taken, then the oral cavities (except for the ulcer parts) of the hamsters were each scraped with an oral swab, and the swab head was cut and put into a centrifuge tube containing a nucleic acid protection liquid. The oral ulcer lesion tissue or normal mucosa was cut and divided into two parts, one part was fixed in 4% paraformaldehyde, and the other part was frozen in liquid nitrogen. The serum was separated from whole blood, and then subpackaged and frozen at -80°C.

### 2. Experimental animal grouping

### Experimental animals in the prevention group, which are divided into the following 10 groups:

| Group | Treatment method |
|---|---|
| Blank group | Normal diet |
| Model group | Normal diet + *Candida albicans* |
| ET-22 living cell group | Normal diet + *Candida albicans + Lactobacillus paracasei* ET-22 live cells |
| ET-22 inactivated cell group | Normal diet + *Candida albicans + Lactobacillus paracasei* ET-22 inactivated cells |
| ET-22 secretion group (extracellular metabolite group) | Normal diet + *Candida albicans + Lactobacillus paracasei* ET-22 secretion |
| K12 living cell group | Normal diet + *Candida albicans + Streptococcus salivarius* K12 live cells |
| L9 living cell group | Normal diet + *Candida albicans + Lactobacillus paracasei* L9 live cells |
| AP32 living cell group | Normal diet + *Candida albicans + Streptococcus salivarius* K12 live cells |
| DSM17938 living cell group | Normal diet + *Candida albicans + Lactobacillus reuteri* DSM17938 live cells |
| HN019 living cell group | Normal diet + *Candida albicans + Bifidobacterium lactis* HN019 |

### Experimental animals in the treatment group, which are divided into the following 7 groups:

| Group | Treatment method |
|---|---|
| Blank group | Normal diet |
| Model group | Normal diet + *Candida albicans* |
| ET-22 living cell group | Normal diet + *Candida albicans + Lactobacillus paracasei* ET-22 live cells |
| ET-22 inactivated cell group | Normal diet + *Candida albicans + Lactobacillus paracasei* ET-22 inactivated cells |
| ET-22 secretion group (extracellular metabolite group) | Normal diet + *Candida albicans + Lactobacillus paracasei* ET-22 secretion |
| K12 living cell group | Normal diet + *Candida albicans + Streptococcus salivarius* K12 live cells |
| L9 living cell group | Normal diet + *Candida albicans + Lactobacillus paracasei* L9 live cells |

### Oral disease infection severity scoring - mice

The mice in each group were euthanized 48 (prevention group)/72 (treatment group) hours after the surgery, and observed for the assessment of the severity of tongue lesions. The macroscopic assessment of infection is expressed by a lesion score of 0 to 4 according to the extent and severity of white curd-like plaques on the tongue surface, as follows: 0 point, normal; 1 point, white plaques below 20%; 2 points, white plaques below 90% but above 21%; 3 points, white plaques above 91%; thick white plaque-like pseudomembranes accounted for above 91%.

The whole tongue of the mouse was longitudinally cut into two parts in the middle of the tongue tip, and one part was fixed in 4% neutral-buffered paraformaldehyde at normal temperature, and then embedded in paraffin. The sample was cut into continuous longitudinal sections with a thickness of 5 µm along the tongue tip and then stained using the hematoxylin method. With reference to literatures, Leica fluorescence microscope was used for staining observation.

### Oral disease infection severity scoring - golden hamsters

After sequential deparaffinization and gradient dehydration of paraffin sections, antigen retrieval was performed using a citric acid antigen retrieval buffer (pH = 6.0), then the sections were incubated in a 3% hydrogen peroxide solution in the dark for 25 min to block endogenous peroxidase, steps of blocking, primary antibody incubation, secondary antibody incubation, etc. were performed, and finally color development with DAB and nuclear counterstaining, dehydration and section mounting were performed for observation and photographing under a microscope. The immunohistochemical score was determined by the IRS (Immunoreactive Score) scoring method. The intensity of staining (0-3 points) and the proportion of positively stained cells (0-4 points) were scored respectively, and then multiplied together to obtain a comprehensive score (0-12 points). The scoring criteria are as follows, in which the intensity of staining is scored on the basis of the staining characteristics exhibited by the proteins of interest: no staining is indicated by a score of 0, light yellow is indicated by a score of 1, yellowish brown is indicated by a score of 2, and brown is indicated by a score of 3; and the proportion of positively stained cells is scored on the basis of the proportion of positive cells in the section: 0-5% positive cells are indicated by a score of 0, 6%-25% positive cells are indicated by a score of 1, 26%-50% positive cells are indicated by a score of 2, 51%-75% positive cells are indicated by a score of 3, and > 75% positive cells are indicated by a score of 4. For the comprehensive score, a score of 0 represents negative (-); a score of 1-3 represents weak positive (+); a score of 4-5 represents positive (+ +); and a score of 6-7 represents strong positive (+ + +).

### Observation results of tissue lesions:

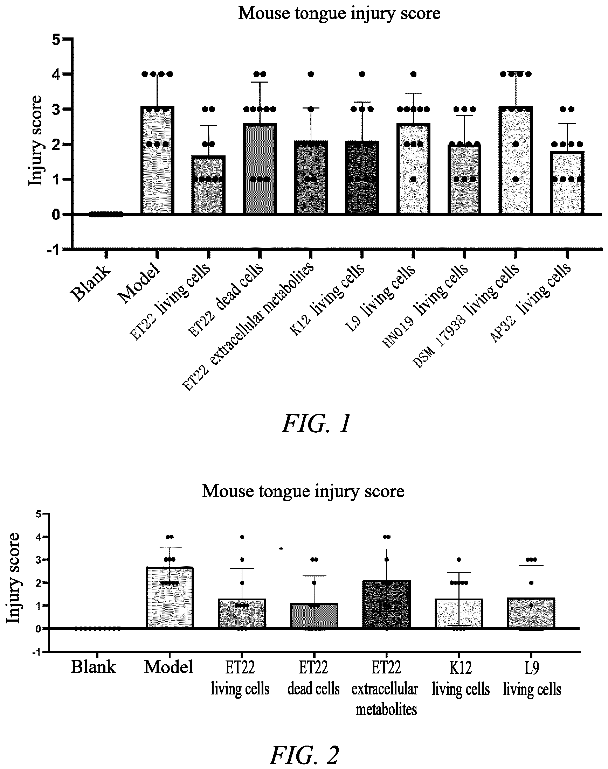

FIG. 1 shows the ICR mouse tongue injury scores 48 h after modeling in the prevention group in Example 2. It can be seen from FIG. 1 that in the prevention group, only the ET-22 living cell group shows a significant difference in mouse tongue injury scores compared with the model group (p < 0.05) 48 hours after the surgery, with a decrease of 46.23% in the mean score; the mean score of the DSM 17938 living cell group is consistent with that of the model group; and injury scores in the remaining experimental groups also decrease to varying degrees, although not significantly different. The injury scores in the ET-22 secretion group (extracellular metabolite group), the K12 living cell group, the HN019 living cell group and the AP32 living cell group decrease by 31.9%, 32.26%, 35.48% and 41.94%, respectively; and the injury scores in the ET-22 inactivated cell group and the L9 living cell group decrease by 16.13% and 16.13%, respectively.

FIG. 2 shows the ICR mouse tongue injury scores 72 h after modeling in the treatment group in Example 2. It can be seen from FIG. 2 that in the treatment group, only the ET-22 inactivated cell group shows a significant difference in mouse tongue injury scores compared with the model group (p < 0.05), while the probiotic groups all show a certain cure rate, in which the metabolites of *Lactobacillus paracasei* ET-22 have the lowest cure rate of 11% and the least injury score decrease of 23%, and the remaining groups have a cure rate of 30%-40% and an injury score decrease of 50%-60%.

Analysis of injury scores in the prevention group:
According to the study of Sanae.A.Ishijima et al., administration of *Streptococcus salivarius* K12 to mouse models, in the model group, the mean tongue injury score is 3.4, and after the treatment with 1.5 × 10⁹ CFU/mL of *Streptococcus salivarius* K12, the mean tongue injury score is 2.0.

In this prevention experiment of the present invention, the mean injury score after the treatment with 1.0 × 10⁹ CFU/mL of *Streptococcus salivarius* K12 is 2.1, and the mean injury scores after the treatment with 1.0 × 10⁹ CFU/mL of *Lactobacillus paracasei* ET-22 live cells, inactivated cells and extracellular metabolite group are 1.7, 2.6 and 2.1, respectively. The *Lactobacillus paracasei* ET-22 live cells are superior to the *Streptococcus salivarius* K12 live cells in preventing candidiasis albicans. The *Lactobacillus paracasei* extracellular metabolite group and the *Streptococcus salivarius* K12 live cells have the same efficacy in preventing candidiasis albicans. Compared with the live cells, the extracellular metabolite group has simpler preservation method and wider application scenarios.

### Observation of tongue dorsum lesion sections:

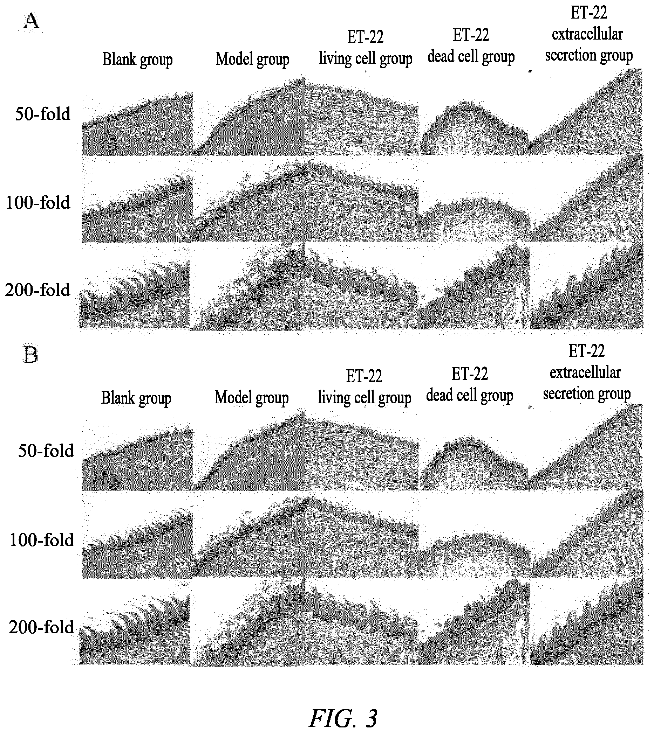

The HE staining of tongue dorsum lesion sections of mice in the blank group, model group and probiotic group 48 h after modeling in the prevention group are as shown in FIG. 3; where the photographs in A show the papillary process of epithelial cells in tongue tissue, and the photographs in B show tissue hyperplasia. It can be seen from FIG. 3 that, compared with the blank group, the model group shows severe inflammatory infiltration, the ET-22 living cell group, ET-22 secretion group (extracellular metabolite group), K12 living cell group, HN019 living cell group and AP32 living cell group show an obvious improvement in inflammatory infiltration, the ET-22 inactivated cell group and the L9 living cell group also show relatively severe inflammatory infiltration, and the DSM 17938 living cell group shows inflammatory infiltration more severe than that in the model group.

It can be seen from FIG. 3 that, compared with the blank group, the model group shows a severe loss of papillary process of epithelial cells in tongue tissue, obvious desquamation, and obvious tissue hyperplasia. The ET-22 living cell group, ET-22 secretion group and AP32 living cell group show well preserved papillary process of epithelial cells in tongue tissue and no desquamation, but show a slight tissue hyperplasia compared with the blank group. The ET-22 inactivated cell group shows a relatively obvious loss of papillary process of epithelial cells in tongue tissue, no desquamation, and a relatively obvious tissue hyperplasia. The L9 living cell group shows a certain loss of papillary process of epithelial cells in tongue tissue, slight desquamation, and tissue hyperplasia similar to that in the model group. The K12 living cell group shows an obvious loss of papillary process of epithelial cells in tongue tissue, no desquamation, and a certain tissue hyperplasia. The DSM 17938 living cell group shows an obvious loss of papillary process of epithelial cells in tongue tissue, slight desquamation, and severe tissue hyperplasia.

It can be seen from the above that the ET-22 living cell group, ET-22 extracellular metabolite group, AP32 living cell group and K12 living cell group have a certain efficacy on the prevention of oral candidiasis, alleviating inflammation and tongue tissue lesions. The ET-22 living cell group has the best therapeutic effect.

### Mucosal tissue sections and HE staining:

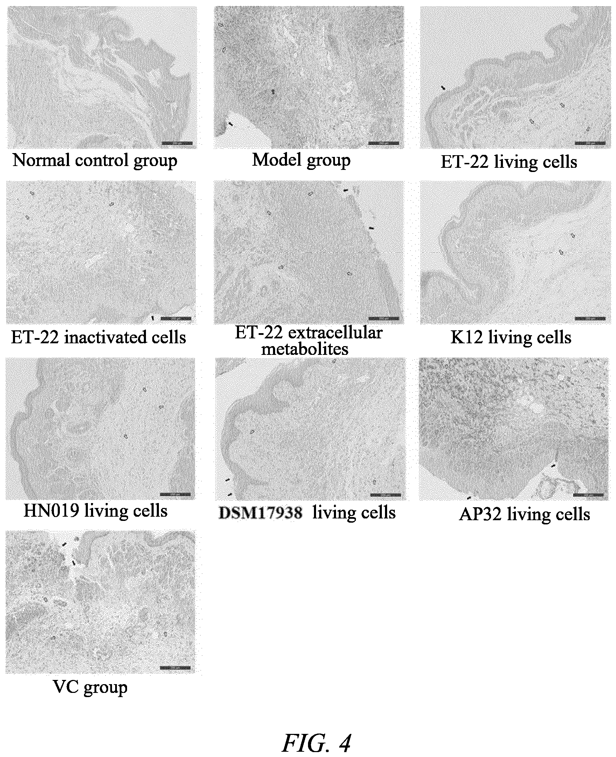

HE staining photographs of typical oral mucosal tissue sections of hamsters 48 h after modeling in the prevention group in Example 2 are as shown in FIG. 4; where white arrows point to inflammatory cell infiltration, black arrows point to damage and shedding of mucosal epithelium, and gray arrows point to capillary filling and dilation and gland hyperplasia (scale bar: 200 µm).As shown in FIG. 4, the oral mucosal epithelium of the hamsters in the normal control group shows intact continuity, normal cell and gland morphology, and no obvious inflammatory cell infiltration. Compared with the oral mucosal epithelium of the hamsters in the normal control group, the oral mucosal epithelium of the hamsters in the model group shows damage, shedding and incomplete continuity, capillary filling and dilation, and a large number of inflammatory cells infiltrated, indicating the successful modeling of the oral ulcer model. Compared with the model group, the ET-22 living cell group shows the best intervention effect, wherein the oral mucosal epithelium of the hamsters in this group shows a relatively intact continuity and obviously decreased inflammatory cells infiltrated; while the oral mucosal epithelium of the hamsters in the ET-22 inactivated cell group and ET-22 extracellular metabolite group shows still partial damage and some inflammatory cells infiltrated. The oral mucosal epithelium of the hamsters in the K12 living cell group and HN019 living cell group shows a relatively high degree of integrity and a small number of inflammatory cells infiltrated; and the oral mucosal epithelium of the hamsters in the DSM17938 living cell group and AP32 living cell group shows an incomplete continuity and obvious inflammatory cell infiltration. Vitamin C is often considered to help prevent and treat oral ulcer. It can be seen from the figure that although the damage of oral mucosal epithelium of the hamsters in the VC group is improved compared with that in the model group, the damage is still relatively severe and there are more inflammatory cells infiltrated. These results indicate that the consumption of ET-22, especially ET-22 living cell, can reduce the onset risk of oral ulcer and alleviate the symptoms of ulcer, and has a better effect than pre-supplementation with VC.

### Immunohistochemical results of mucosal tissues:

NF-κB is an important transcription factor, and the activation of its signaling pathway can initiate inflammatory responses; in addition, the activation of NF-κB signaling pathway can promote the upregulation of the expression of MMP-9, and MMP-9 can degrade laminin and type IV collagen, thereby accelerating the infiltration and transfer of inflammatory cells. The high expression of Caspase 3 may promote the upregulation of apoptosis and hydrolysis of PARP, resulting in a failure of PARP to participate in DNA damage repair normally, thus aggravating apoptosis.

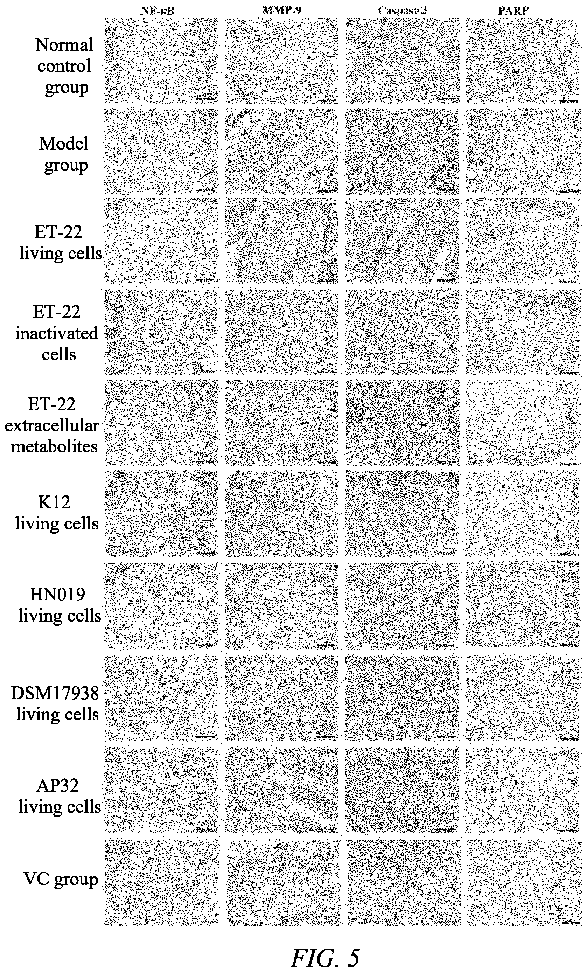

The immunohistochemical photographs of typical oral ulcer mucosal tissues of hamsters 48 h after modeling in the prevention group (scale bar: 100 µm) are as shown in FIG. 5. As shown in FIG. 5 and Table 1, the oral mucosal tissue of the hamsters in the normal control group shows negative expression of NF-κB, MMP-9, Caspase 3 and PARP, while the expression of the above four proteins significantly increases in the model group (*P* < 0.05). The expression of NF-κB and MMP-9 in the model group significantly increases, indicating that an inflammatory response occurs in the oral mucosa and the connection between the basement membrane and cells is disrupted. The expression of Caspase 3 and PARP in the model group significantly increases, indicating that the mucosal apoptosis is upregulated, triggering epithelial damage and shedding of ulcerated mucosa. The positive expression of these proteins in the model group is consistent with the results reflected by HE staining.

Compared with the model group, the ET-22 living cell group, ET-22 inactivated cell group, ET-22 extracellular metabolite group and K12 living cell group all show a significant decrease in the expression of these four proteins (*P* < 0.05); the HN019 living cell group shows a significant decrease in the expression of these proteins except for NF-κB; the DSM17938 living cell group shows a significant decrease only in the expression of Caspase 3 and PARP, with no significant difference from the model group in the expression of NF-κB and MMP-9; the AP32 living cell group shows a significant decrease in the expression of NF-κB, Caspase 3 and PARP, with no significant difference from the model group in the expression of MMP-9; and the VC group shows a significant decrease only in the expression of NF-κB and Caspase 3, and downregulation of the expression of MMP-9 and PARP, but with no significant difference from the model group in the expression of MMP-9 and PARP (Table 1). The results show that ET-22 has a positive effect of preventing oral ulcer.

**Table 1 Mean immunohistochemical score of oral mucosal tissues**

| Group | NF-xB | MMP-9 | Caspase 3 | PARP |
|---|---|---|---|---|
| Normal control group | 0 (-)^{d} | 0 (-)^{d} | 0 (-)^{b} | 0 (-)^{d} |
| Model group | 5 (++)^{a} | 3 (+)^{a} | 3 (+)^{a} | 7 (+++)^{a} |
| ET-22 live cells | 1 (+)^{cd} | 1 (+)^{bcd} | 1 (+)^{b} | 2 (+)^{cd} |
| ET-22 inactivated cells | 2 (+)^{bcd} | 1 (+)^{bcd} | 1 (+)^{b} | 2 (+)^{cd} |
| ET-22 extracellular metabolites | 2 (+)^{bcd} | 1 (+)^{cd} | 1 (+)^{b} | 2 (+)^{cd} |
| K12 live cells | 1 (+)^{cd} | 1 (+)^{cd} | 0 (-)^{b} | 2 (+)^{cd} |
| HN019 live cells | 3 (+)^{abc} | 1 (+)^{bcd} | 1 (+)^{b} | 4 (++)^{bc} |
| DSM17938 live cells | 4 (++)^{ab} | 3 (+)^{ab} | 1 (+)^{b} | 2 (+)^{cd} |
| AP32 live cells | 1 (+)^{cd} | 3 (+)^{a} | 1 (+)^{b} | 2 (+)^{cd} |
| VC group | 2 (+)^{bcd} | 2 (+)^{abc} | 1 (+)^{b} | 5 (++)^{ab} |

### The levels of lipoxin A4 (LXA4) and prostaglandin E2 (PGE2) in oral ulcer mucosal tissues:

LXA4 is an endogenous lipid with an anti-inflammatory effect that inhibits the secretion of pro-inflammatory cytokines such as IL-6.

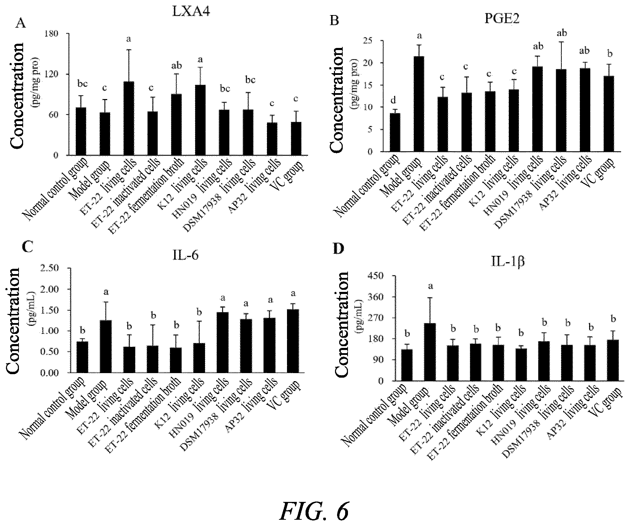

The levels of LXA4 and PGE2 levels in the oral ulcer mucosal tissue of hamsters are as shown in FIG. 6. FIG. 6 shows that there is no significant change in the content of LXA4 in the model group compared with that in the normal control group, speculating that because the model group is not subjected to intervention, the degree of LXA4 activation is low and the inflammation resolves slowly, thus also resulting in more severe and long-lasting ulcer. Compared with the model group, the ET-22 living cell group, ET-22 extracellular metabolite group and K12 living cell group have a significant increase in the content of LXA4 (*P* < 0.05), indicating that the anti-inflammatory role in these groups can be played by increasing the level of LXA4, thereby alleviating the symptoms of oral ulcer; and the ET-22 inactivated cell group, HN019 living cell group, DSM17938 living cell group, AP32 living cell group and VC group are not significantly different from the model group.

PGE2 can exacerbate the inflammatory response, promote local vasodilation and increase capillary permeability. Compared with the normal control group, the model group has a significant increase in the content of PGE2, indicating a severe inflammation of the oral mucosa in the model group, consistent with its severe ulcer phenotype. Compared with the model group, the ET-22 living cell group, ET-22 inactivated cell group, ET-22 extracellular metabolite group, K12 living cell group and VC group all have a significant decrease in the content of PGE2 (*P* < 0.05), with the level between that in the model group and that in the control group; while the HN019 living cell group, DSM17938 living cell group and AP32 living cell group are not significant different from the model group.

### The levels of pro-inflammatory cytokines in sera:

The levels of pro-inflammatory cytokines IL-6 and IL-1β in the sera of hamsters are as shown in FIG. 6. Compared with the normal control group, the model group has a significant increase in the levels of pro-inflammatory cytokines IL-6 and IL-1β in the sera of hamsters (P < 0.05, FIG. 6), indicating the successful modeling of the oral ulcer model. The ET-22 living cell group, ET-22 inactivated cell group, ET-22 extracellular metabolite group and K12 living cell group all have a significant decrease in the concentration of IL-6 in the sera of hamsters compared with the model group (P < 0.05), indicating that ET-22 and K12 can reduce the inflammatory response, thereby slowing down oral ulcer; in addition, all intervention groups all have a significant decrease in the concentration of IL-1β in the sera of hamsters compared with the model group (P < 0.05). The results suggest that ET-22 and K12 have a better immunomodulatory effect than other groups and can resist the formation or deterioration of oral ulcer by regulating the body's immunity.

### The SOD activity, MDA concentration and GSH concentration in sera

SOD and GSH are important antioxidases and antioxidants, and the increase in SOD and GSH indicates that the body produces oxidative stress to stimulate the increase in antioxidant substances; and MDA is a product of lipid peroxidation, reflecting the degree of damage caused by oxidative stress.

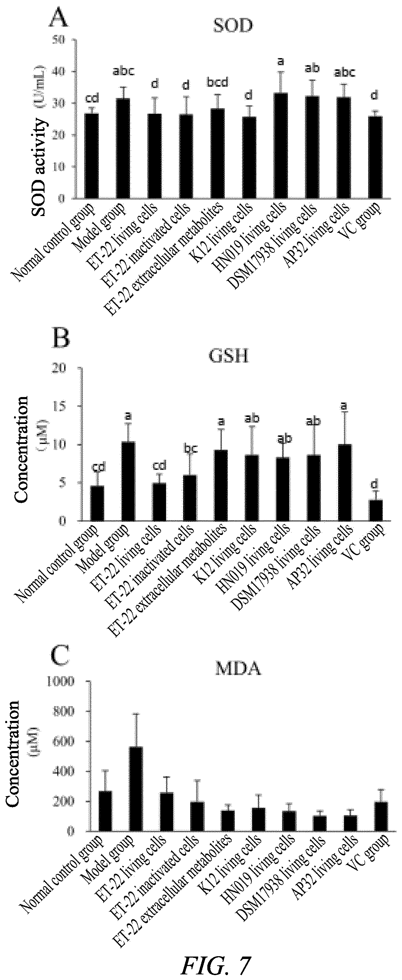

48 h after modeling in the prevention group, the SOD activity, and the GSH and MDA levels in the sera of hamsters are as shown in FIG. 7. Compared with the normal control group, the model group has a significant increase in the levels of GSH and MDA in the sera of hamsters (P < 0.05), and also has an increase in the SOD activity, but not a significant increase. Compared with the model group, the ET-22 living cell group, ET-22 inactivated cell group and VC group all have a significant decrease in the SOD activity, and GSH and MDA levels (P < 0.05), and the ET-22 extracellular metabolite group has a significant decrease only in the level of MDA. In each of the remaining control strain groups, the level of MDA significantly decreases, but no obvious regulatory effect is seen in the SOD activity and the GSH level. The results of the VC group are expected and consistent with the antioxidant properties of VC. The results of the ET-22 living cell group and ET-22 inactivated cell group show that they have similar effects to VC, and can reduce damage by oxidative stress to a certain degree and have good antioxidant potential.

### Diversity and composition of oral flora

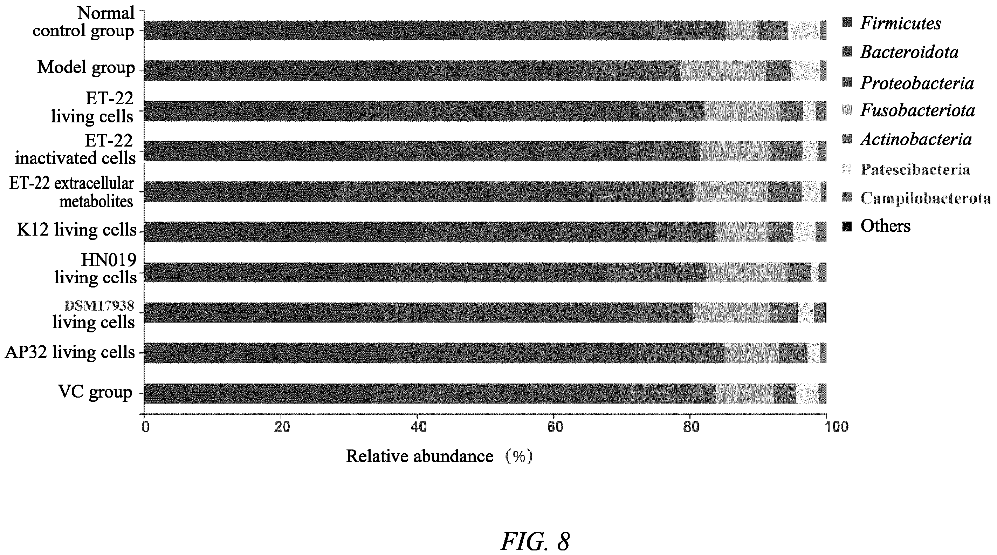

48 h after modeling in the prevention group, the composition of oral bacteria in hamsters at the phylum level is as shown in FIG. 8. From the compositional analysis at the phylum level (FIG. 8), it can be seen that the core flora of the hamster oral cavity includes *Firmicutes, Bacteroidota, Proteobacteria, Fusobacteriota,* etc., consistent with previous reports.

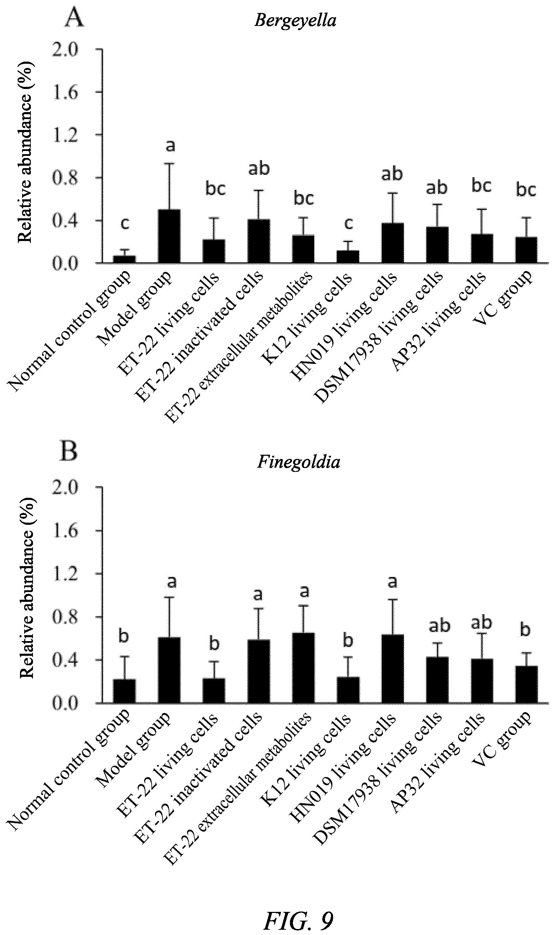

48 h after modeling in the prevention group, the bacterial genera with significant differences in the oral cavity of hamsters are as shown in FIG. 9. From the analysis at the generic level (FIG. 9), it is found that the relative abundance of *Bergeyella* and *Finegoldia* in the oral cavity of hamsters in the model group is significantly higher than that in the normal control group (P < 0.05). *Bergeyella* have been found to be closely associated with oral diseases such as periodontal disease; and *Finegoldia* can induce inflammation by activating neutrophils. Compared with the model group, the ET-22 living cell group has a significant downregulation in the proportion of *Bergeyella* and *Finegoldia,* the ET-22 extracellular metabolites can significantly downregulate the proportion of *Bergeyella* (P < 0.05), while the ET-22 inactivated cell group shows no significant change. Therefore, the ET-22 live cells can reduce the onset risk of oral ulcer by inhibiting harmful bacteria in the oral cavity.

### Example 3

In this example, the effects of the *Lactobacillus paracasei* ET-22, ET-22 extracellular metabolites, and *Streptococcus salivarius* K12 on the biofilm production and biofilm structure of *Streptococcus mutans* are compared.

Construction of saliva-coated hydroxyapatite model: A mixture of a staining liquid and a bacterial liquid was prepared for later use, wherein the bacterial liquid used a *Lactobacillus paracasei* ET-22 living cell solution, ET-22 extracellular metabolite solution, and *Streptococcus salivarius* K12 solution, the concentration of the bacterial liquid was 10⁹ CFU/ml, and the concentration of the extracellular metabolite solution was the equivalent bacterial concentration. Hydroxyapatite (HA) beads were accurately taken and subjected to autoclaved sterilization, and then placed in a 24-well culture plate, 1.5 ml of artificial saliva containing 0.2% sucrose was added to each well, the beads were soaked at 37°C for coating and culture, and then washed twice with 1.5 ml of sterile PBS, the buffer was sucked out, 750 ml of each of the bacterial suspensions was added, and culture was performed for 2 h at 37°C (during which different time periods were selected for performing measurement, and rinsing was performed for 10 s with 1.5 ml of sterile PBS before each measurement, a total of three times) to prepare the saliva-coated hydroxyapatite model that simulates the saliva acquired pellicle structure on the tooth surface. By crystal violet staining, the results measured at 595 nm using a microplate reader were used to characterize the biofilm production. Detection was performed using a fluorescence bioimaging system. To quantify the staining of bacterial biofilms and bacterial colonization on the HA model, the fluorescence intensity thresholds for each fluorescent color were manually set, and the fluorescence intensity analysis for the fluorescence imaging was performed using the Spectral instruments imaging software program.

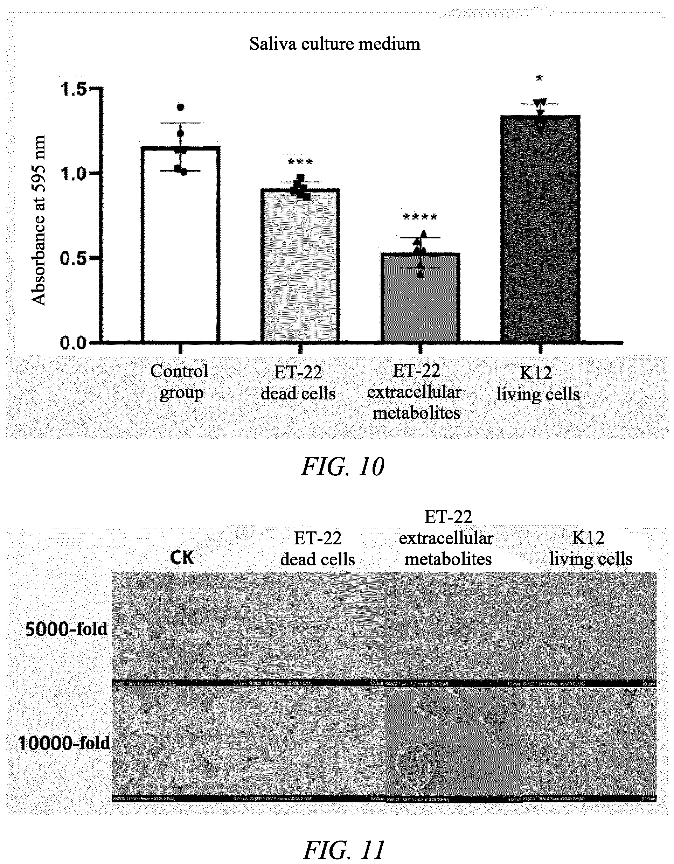

FIG. 10 shows the changes in the biomass of the simulated tooth surface biofilms under the intervention of various probiotic groups in a simulated saliva environment. It can be seen from FIG. 10 that, the biofilm production in the ET-22 inactivated cell group and extracellular metabolite group significantly decreases, and the difference is extremely significant (P < 0.00001). In comparison, the biofilm production in the ET-22 extracellular metabolite group is the lowest; and the biofilm production in the K12 living cell group is higher than that in the control group.

In this example, the changes in the biomass structure of the simulated tooth surface biofilms under the intervention of various probiotic groups in a simulated saliva environment are analyzed using SEM. FIG. 11 shows the changes in the biomass structure of simulated tooth surface biofilms under different probiotic interventions by scanning electron microscope in Example 3. As shown in FIG. 11, in the artificial saliva culture system control group, a cauliflower-like multilayer stacked biofilm three-dimensional structure is formed; in the ET-22 inactivated cell group and the extracellular metabolite group, the content of the biofilm is significantly inhibited, and the cauliflower-like biofilm structure is destroyed; and in the K12 living cell group, a single-layer stacked biofilm state is present, and the inhibitory effect is not significant. Among them, the ET-22 extracellular metabolite group shows the most obvious inhibitory effect, with biofilm showing a monolayer and scattered distribution, which is significantly better than the other treatment groups.

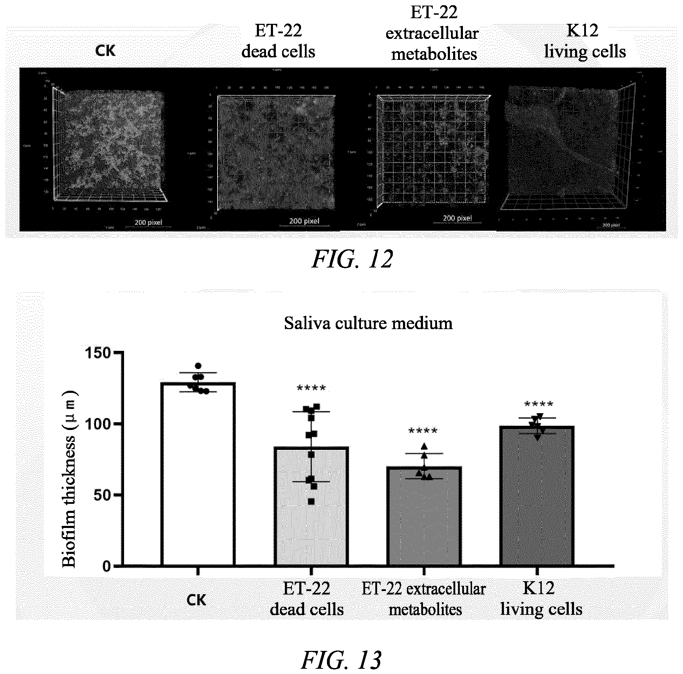

In this example, the changes in the biomass structure and thickness of the simulated tooth surface biofilms under the intervention of various probiotic groups in a simulated saliva environment are analyzed using a confocal laser scanning microscope. The changes in the structure are as shown in FIG. 12. The content of cells in the biofilm in the control group is dominated by live cells (green). The content of dead cells (red) in the biofilm in the ET-22 inactivated cell group increases significantly. The ET-22 extracellular metabolites significantly reduce the number of live cells in the biofilm while reducing the distribution area of the biofilm. The changes in the thickness of the biofilms are as shown in FIG. 13. The thickness of the biofilm in the control group is as high as 120 µm. Both the ET-22 inactivated cell group and the extracellular metabolite group show significantly inhibited thickness of the biofilm, and under the treatment with the ET-22 extracellular metabolites, the mean thickness of the biofilm is only 62 µm, with a decrease of 48% compared with that in the control group.

Finally, it should be stated that the above are merely used for illustrating rather than limiting the technical solution of the present invention; and although the present invention has been described in detail with reference to the preferred embodiments, those of ordinary skill in the art should understand that modifications or equivalent substitutions may be made to the technical solution of the present invention without departing from the spirit and scope of the technical solution of the present invention.

## Claims

1. Use of *Lactobacillus paracasei* in preparing a medicament for preventing and/or treating oral ulcer, **characterized in that** the *Lactobacillus paracasei* comprises *Lactobacillus paracasei* ET-22 cells and/or *Lactobacillus paracasei* ET-22 extracellular metabolites, and the *Lactobacillus paracasei* ET-22 is a strain with deposition number CGMCC No. 15077.

2. The use according to claim 1, **characterized in that** the *Lactobacillus paracasei* ET-22 cells are *Lactobacillus paracasei* ET-22 live cells and/or *Lactobacillus paracasei* ET-22 inactivated cells; and
the *Lactobacillus paracasei* ET-22 extracellular metabolites are prepared according to the following method as:
removing the bacterial cells of *Lactobacillus paracasei* ET-22 from its incubation solution to obtain the *Lactobacillus paracasei* ET-22 extracellular metabolites; preferably, the incubation solution is obtained by incubating the *Lactobacillus paracasei* ET-22 in water.

3. The use according to claim 1 or 2, **characterized in that** the oral ulcer includes oral ulcer caused by pathogenic bacteria, oral ulcer caused by oxidative damage, or oral ulcer caused by immunocompromise.

4. A probiotic composition, **characterized by** comprising: *Lactobacillus paracasei* ET-22 cells and/or *Lactobacillus paracasei* ET-22 extracellular metabolites, wherein the *Lactobacillus paracasei* ET-22 is a strain with deposition number CGMCC No. 15077.

5. The probiotic composition according to claim 4, further **characterized by** comprising: sodium carboxymethyl cellulose, propolis, borneol, a lubricant, glycerol, and Tween-20,
wherein preferably, the probiotic composition comprises the following components in parts by weight:
15-20 parts, for example, 18-20 parts of *Lactobacillus paracasei* ET-22 cells and/or *Lactobacillus paracasei* ET-22 extracellular metabolites;
10-20 parts, for example, 14-20 parts of sodium carboxymethyl cellulose;
20-40 parts, for example, 25-35 parts of propolis;
2-5 parts of borneol;
2-5 parts of lubricant;
8-12 parts of glycerol; and
1-3 parts of Tween-20.

6. The probiotic composition according to claim 4 or 5, **characterized in that** the *Lactobacillus paracasei* ET-22 cells are *Lactobacillus paracasei* ET-22 live cells and/or *Lactobacillus paracasei* ET-22 inactivated cells; and
the *Lactobacillus paracasei* ET-22 extracellular metabolites are prepared according to the following method:
removing the bacterial cells of *Lactobacillus paracasei* ET-22 from its incubation solution to obtain the *Lactobacillus paracasei* ET-22 extracellular metabolites; preferably, the incubation solution is obtained by incubating the *Lactobacillus paracasei* ET-22 in water.

7. Use of the composition according to any one of claims 4-6 in preparing a composition for preventing and/or treating oral ulcer,
**characterized in that** preferably, the oral ulcer includes oral ulcer caused by pathogenic bacteria, oral ulcer caused by oxidative damage, or oral ulcer caused by immunocompromise.

8. The use according to claim 7, **characterized in that** the composition for preventing and/or treating oral ulcer is a pharmaceutical composition; preferably, the pharmaceutical composition is a patch.

9. A method for preparing a probiotic patch, **characterized by** comprising:
(1) a step of uniformly mixing *Lactobacillus paracasei* ET-22 cells and/or *Lactobacillus paracasei* ET-22 extracellular metabolites, propolis, sodium carboxymethyl cellulose and a lubricant to obtain a mixed system A;
(2) a step of uniformly mixing borneol, glycerol and Tween-20 under heating in a water bath to obtain a mixed system B;
(3) a step of uniformly mixing the mixed system A and the mixed system B; and
(4) a step of performing drying treatment.

10. A probiotic patch, **characterized in that** the probiotic patch is prepared from the composition according to any one of claims 4-6, or prepared by the method according to claim 9.

11. A method for treating oral ulcer, **characterized by** comprising administering to a subject an effective amount of the composition according to any one of claims 4-6 and/or the probiotic patch according to claim 10, wherein the subject includes a mammal or a human.
